# EUROPEAN PATENT APPLICATION

(11) **EP 2 023 255 A1**
(43) Date of publication of application: **11.02.2009**
(21) Application number: 07015407.5
(22) Date of filing: 06.08.2007
(51) Int. Cl.: G06F 19/00

(54) **Method for the evaluation and selection of pharmaceutical compounds**

(71) Applicant: Lampe, Johannes, Dr. med. habil., 13465 Berlin (DE)
(72) Inventor: Lampe, Johannes, Dr. med. habil., 13465 Berlin (DE)
(74) Representative: Grünecker, Kinkeldey, Stockmair & Schwanhäusser Anwaltssozietät

(57) **Abstract**

The present invention refers to a method for evaluating, comparing and selecting entities over a broad variety of technical fields. Preferably, the entities are pharmaceutical drugs.

## Description

### Field of the Invention

The present invention refers to a method for evaluating, comparing and selecting entities over a broad variety of technical fields. Preferably, the entities are pharmaceutical drugs.

### Background of the invention

In pharmaceutical drug discovery and development, one out of ten compounds fails. It is estimated that the development of one product ready to enter the market costs about 800 Million $. Late stage failure leads to extremely high costs, since especially phase II and phase III trials are very expensive. Different reasons are leading to the high attrition rate in discovery and development: Clinical safety and efficacy, formulation, PK/bioavailability, commercial reasons, toxicology, cost of goods, and other reasons (Frank and Hargreaves, 2003).

In this context the term compound is used for (1) any substance used for diagnostic, therapeutic and prophylactic purposes consisting out of natural or synthetically produced and where applicable (pharmaceutically) specifically manufactured active ingredients. Additionally, the term compound is used for (2) suture material, disinfectants, diagnostics and several assistive technologies, e.g. pacemaker and contact lenses.

The portfolio management process can be divided into three sub-categories. The corporate portfolio management is supporting senior management to take informed decisions managing the approved drugs and those in development to serve an optimal corporate performance. Both the R&D and marketing portfolio management are feeding into the corporate portfolio management with a technical and commercial evaluation respectively. Independently, they are viewed as essential in themselves (Seget, 2003). There are several databases available which provide comprehensive technical and commercial evaluations for the assessment of the R&D and marketing portfolio management. However, these databases only provide rather unprocessed and often poorly sorted data in form of complex reports on single compounds. Other sources of information on a compound are derived from experimental and clinical data and can be found in primary scientific publications.

During the technical and commercial evaluation of a compound, huge amounts of information have to be collected and processed. Different groups contribute to this process such as global development teams and specialized sub-teams (e.g. pharmacokinetics, toxicology, clinical trial design). The results of those analyses are presented in form of 20 to 30 pages of an expert report. In order to achieve comprehension and brevity the multi-facetted aspects of the evaluation process are often reduced to simplifying summaries. As a consequence, existing knowledge is not optimally used, important information is lost and opportunities may be missed (Rooney et al., 2001).

Additionally, academic and industrial consultants take part in the evaluation of compounds depending on the field of expertise they cover (e.g. medical, animal models). The approach of those consultants is comparable to the way R&D project team experts work.

Some solution providers deliver software, which may help to collect as many data as possible on a specific compound and make it easily retrievable (e.g. Pipeline pilot™). Noteworthy, these approaches only support the organization of complex data sets and therefore only indirectly impact on the decision making process.

Other consulting firms focus more on specific aspects of drug development, especially on pharmacokinetic and pharmacodynamic issues. These approaches concentrate on modelling and simulations of clinical trials (Rooney et al., 2001; Poland and Wada, 2001; Hunt et al., 1998) or the prediction of the outcome of clinical phase III trials (De Ridder, 2005). The successful application of such pharmacokinetic/pharmacodynamic (PK/PD) modelling approaches may be one important reason why PK / bioavailability issues as cause of compound failure dropped from >40 percent to about 10 percent between 1991 and 2000. However, there are other reasons for the failure of drugs in R&D, which are not sufficiently covered, yet. Especially, in about 40 percent of all cases compounds fail due to the lack of efficacy (∼ 30%) or safety (∼ 10%) with no significant change since 1991 (Frank and Hargreaves, 2003).

In this context "efficacy" is the therapeutic effect of a drug on a disease and "safety" refers to the side effects of the drug on the organism.

To address these important issues Schachter and Ramoni use various pre-defined variables to predict the outcome of a drug (Schachter et al., 2005; Schachter and Ramoni, 2007). However, for this Bayesian mathematics-based approach the authors need to include a mathematical assumption (high vs. low likelihood to succeed), which may introduce arbitrary, non-technical aspects in the results obtained. Furthermore, they compare the compound to be evaluated with a compound class instead of single compounds.

### Literature

Anonymous (2007a). International Standard Industrial Classification ISIC Rev.4 (draft) structure (United Nations Statistics Division; status as of July 2007). Available from World Wide Web: http://unstats.un.org/unsd/cr/registry/regcst.asp?Cl=27.
Anonymous (2007b). The Merck Manual Online medical library (Editors: Porter, R.S.; Kaplan J.L.; status as of May 2007). Available from World Wide Web: http://www.merck.com/mmpe/index.html.
Bard, J.B.L. and Rhee S.Y. (2004). Ontologies in biology: Design, applications and future challenges. Nature Reviews Genetics (5): 212 - 220.
Barabási, A.-L and Oltvai Z.N. (2004). Network Biology: Understanding the cell's functional organization. Nature Reviews Genetics (5): 101-113.
De Ridder, F. (2005). Predicting the outcome of phase III trials using phase II data: a case study of clinical trial simulation in late stage drug development. Basic Clin. Pharmacol. Toxicol. 96: 235-241.
Frank, R. and Hargreaves, R. (2003). Clinical biomarkers in drug discovery and development. Nat Rev Drug Discov. 2(7):566-80.
Hunt, C.A.; Guzy, S. and Weiner, D.L. (1998). A forecasting approach to accelerate drug development. Stat. Med.: 17, 1725-1740.
Imming, P., Sinning C. , and Meyer A. (2006). Drugs, their targets and the nature and number of drug targets. Nat Rev Drug Discov. 2006 Oct;5(10):821-34.
Kirschner, M.W. (2005). The Meaning of Systems Biology. Cell, Vol. 121, 503-504, May 20, 2005.
Newman, M.E.J. (2003). The Structure and Function of Complex Network. Siam Review (45): 167-256.
Poland, B. and Wada, R. (2001). Combining drug-disease and economic modelling to inform drug development decisions. Drug Discov. Today 6, 1165-1170.
Rooney, K.F.; Snoeck, E. and Watson P.H. (2001). Modelling and simulation in clinical drug development. Drug Discov Today: 6(15):802-806.
Schachter, A.D., Needham M.A. und Ramoni, M.F. (2005). Method and Apparatus for evaluating chemical entities. US Patent 2005/0021237 A1.
Schachter AD, Ramoni MF (2007). Clinical forecasting in drug development. Nat Rev Drug Discov.:107-8.
Seget, S. (2003). The Pharmaceutical Portfolio Management OutL∞k: Integrated decisionmaking in marketing and R&D. Reuters Report

### Summary of the invention

One object underlying the present invention is to overcome the disadvantages of these prior art methods and provide an improved means for comparing and selecting pharmaceutical drugs.

Another object underlying the present invention is to increase "safety" and/or "efficacy" of pharmaceutical drug candidates.

Another object underlying the present invention is to minimize costs in pharmaceutical drug discovery and development.

Another object underlying the present invention is to provide a universal means which assists in comparing and selecting most promising entities from a collective of candidate entities.

These objects are solved by providing a method for comparing and evaluating candidates in a class of compounds or entities (herein also denominated "compounds"), which method ("Evaluation Tool") comprises:
a) Performing compound profiling by defining various categories and assigning plural defined categories to individual compounds or entities and characterizing and evaluating compounds or entities according to said categories;
b) Performing compound benchmarking by comparing results within the same category of one compound with results of the same category of other compounds or entities and generating compound benchmarking for different categories based on the outcome of the comparison;
c) Generating a benchmark representation including combining different compound benchmarks for different categories.

In a preferred embodiment, the method for comparing and evaluating candidates in a class of compounds or entities is one
wherein step a) of the method above comprises:
α) Collecting data d₁(i), d₂(i), ...dₙ(i), with n = 1 ... n = ∞ for each member mᵢ of a class of compounds or entities C(mᵢ), with C(mᵢ) and 0< i< ∞;
β) Putting data d₁(i), d₂(i), ... dₙ(i) into different categories S₁(i), S₂(i), .... Sₙ(i), with n = 1 ... n= ∞, wherein
   β1) for each member mᵢ among the categories S₁(i), S₂(i), .... Sₙ(i) particular categories are selected which are sorted under the first level of hierarchy L₁ according to the taxonomy of hierarchy L₁, wherein taxonomy is the sorting of categories and sub-categories L₁, L₂, ... L_{∞} below a particular L₁ category and all categories below a particular L₁ level category conceptually belong to this L₁ category;
   β2) for each member mᵢ among the remaining categories S₁(i), S₂(i), .... Sₙ(i) categories are selected, which are sorted under the other levels of hierarchy sub-levels L₂,L₃ ... L_{∞} according to the taxonomy of the respective level L₂, L₃ ... L_{∞};
   β3) for each member mᵢ among the still remaining categories, which cannot be sorted based on the taxonomy are correlated with the overall success or failure of the compound or entity to be evaluated and are grouped into additional sub-levels L₂,L₃ ... L_{∞}, wherein
   overall success or overall failure is defined as a function of all members m_{¡} of a class of compounds or entities C(mᵢ), with C(mᵢ) and 0< i< ∞, which fulfil particular predefined criteria for success or failure,
   overall success is defined as the achievement of a predefined objective or goal, and
   overall failure refers to the state or condition of not meeting predefined objective or goal and is defined as the opposite of success, and
   for the data for each member mᵢ and each first Level L₁ a ranking Rᵢ,L₁ is established as follows: Rᵢ,L₁ = {dj(L₁), dk(L₂), dl(L₂), ...}, with j,k,j are integers equal to or greater than 1;
   wherein step b) of the method above comprises:
γ) Comparing or sorting the members mᵢ of the class of compounds or entities C(mᵢ), comprising:
   γ1) determining a probability of success p₁(m_{¡}) for each member mᵢ of a class of compounds or entities which is a function of all dᵢ(L₁) of the first level L₁ hierarchy;
   γ2) determining further probabilities of success pₓ(mᵢ) for each member mᵢ of a class of compounds or entities which is a function of all dᵢ(Lₓ) of the x-^{th} level L₁ hierarchy;
   γ3) comparing each member mᵢ of the class of compounds or entities C(mᵢ) with each other member mⱼ of the class of compounds or entities C(mᵢ) within the same L₁, L₂, L₃... L_{∞} by comparing probabilities pₓ(mᵢ) and pₓ(mⱼ);
   γ4) optionally sorting all members mᵢ based on the order of probabilities p₁ and pₓ, wherein the probabilities p₁ are used in a first sorting step, the probabilities pₓ are used in a x-^{th} sorting step, wherein if there are more than one data and probabilities of the first level L₁ for each ranking R a sorting step is performed;
δ) Comparing all data d₁(i), d₂(i), ...dₙ(i) of a compound or entity mᵢ within the same L₁, L₂,L₃... L_{∞} as a function of Rᵢ,L₁ = {dj(L₁), dk(L₂), dl(L₂), ...}, which means a comparison of each compound with each other compound regarding the same category S₁(i), S₂(i), .... Sₙ(i) is enabled, wherein the combination of different R_{¡},L₁ = {dj(L₁), dk(L₂), dl(L₃), ...} in different L₁, L₂,L₃...L_{∞} enables to characterize the various facets of a compound or entity, wherein
   δ1) the probability of success or failure is calculated for each category S₁(i), S₂(i), S₃(i), ... S_{∞} within L₁ regarding each member mᵢ of a class of compounds or entities C(mᵢ), with C(mᵢ) and 0< i< ∞ and compound profiling is the combination of different probability of success or failure for each member mᵢ of a class of compounds or entities C(mᵢ), with C(mᵢ) and 0< i< ∞ at each L₁, respectively;
   δ2) additional information can be added by calculating the probability of success or failure for each category S₁(i), S₂(i), Sₙ(i) at each level L₂,L3 ... L∞; and δ3) additional information may be added by calculating the probability of success or failure for the combination of different categories S₁(i), S₂(i), .... Sₙ(i) below different L₁, L₂,L₃ ... L_{∞};
ε) Optionally selecting a optimal member mₘₐₓ out of the class of compounds or entities C(mᵢ) based on the basis of one or more of the probabilities of success obtained in steps δ1) and/or δ2) and/or δ3), and
   optionally providing a row of members m_{sec}, m_{third}, ... out of the class of compounds or entities C(mᵢ) based on the basis of one or more of the probabilities of success obtained in steps δ1) and/or δ2) and/or δ3), wherein m_{sec}, m_{third}, ... are the second and third, ... optimal members; and
ζ) Identifying the possibility to improve the probability of success / reduce the probability of failure by adding additional data to each member mᵢ of a class of compounds or entities C(mᵢ), with C(mᵢ) and 0< i< ∞.

The unique features of the method of the present invention ("Evaluation Tool") help to gather comprehensive data on numerous compounds and different indications to address the above-mentioned safety and efficacy issues (besides others).

The invention makes use of the "compound profiling" approach, which enables the user to collect data, both in hypothesis-free and hypothesis-driven ways. Data regarding entities or compounds, e.g. drugs (or fruits, see example 1) can be put in different categories (in the fruit example boxes, s. below) L₁, L₂, L₃ ... . This is a "hypothesis-driven approach" since the level for each box is pre-defined.

On the other hand, each box can be evaluated with regards to its impact on the overall success (or other aspects of the compound) separately (i.e. without .looking at the respective hierarchy level). This is a "hypothesis-free approach".

In both cases, however, the compound to be evaluated is characterized from different perspectives, which is called "compound profiling".

The "compound benchmarking" approach allows comparing the compound under evaluation with similar and different compounds, which have been successfully developed or failed in the same or different indications or at a distinct step in the discovery and development process.

"Compound benchmarks" are particular results within a category.

In a preferred embodiment "results" are values of the categories. These values may be qualities such as figures, letters or marks, or quantities such as parameters to be measured, preferable measured in SI units. These values are assignable to a compound or entity.

Examples for quantities are:
- Area under the curve (AUC)
- Bioavailability
- Half life of the compound (t1/2)
- Interval between two time-points of drug administration
- Number of clinical trials with the compound under evaluation in pathophysiologically related indications
- number of clinical trials of the compound under evaluation in pathophysiologically un-related indications

Examples for qualities are:
- Different classifications for various types of targets (e.g. Imming et al., 2006)
- Different classifications of drugs (e.g. Agonist, Antagonist, partial Agonist)
- Status of clinical trial(s) with compound(s) binding to the same target as the compound under evaluation (e.g. compound launched, registered, pre-registration, successful phase III study, phase III study prematurely terminated, ...)
- Status of clinical trial(s) of compound(s) utilizing the same mode of action as the compound under evaluation (e.g. compound launched, registered, pre-registration, successful phase III study, phase III study prematurely terminated, ...)

In a preferred embodiment "compound benchmarks" are particular boundary values of the categories. These values are defined as described above.

In another preferred embodiment "compound benchmarks" are particular qualities, which are assignable to a compound or entity.

As illustrated above, there are several approaches, which may increase the quality of compound assessment and may help to better predict the outcome of drugs in development. Several of the advantages of these approaches are also incorporated in the method of the present invention ("Evaluation Tool"):
The data may be collected in an independent, and highly standardized way. The standardization allows the data to be put in the database following pre-defined rules. This leads to an objective collection of data and un-biased data entry. In the case of example 1 below, fruits are evaluated (put into different boxes) in a standardized, pre-defined way i.e. the boxes are pre-defined and there are clear rules how the boxes will be filled (e.g. it is clear which box is for which colour). Therefore, the data can be flexibly combined which allows the integration of data for different purposes (e.g. R&D/Marketing portfolio data integration, Marketing/Corporate portfolio data integration, Corporate/R&D portfolio data integration) (Seget, 2003).

This process is further supported by the fact that the data can be visualized in various ways. This allows to easily identify and assess relevant data and critical issues allowing for an informed decision-making.

The novel combination of the features according to the method of the present invention make the "Evaluation Tool" a major help for evaluating compounds.

### List of figures

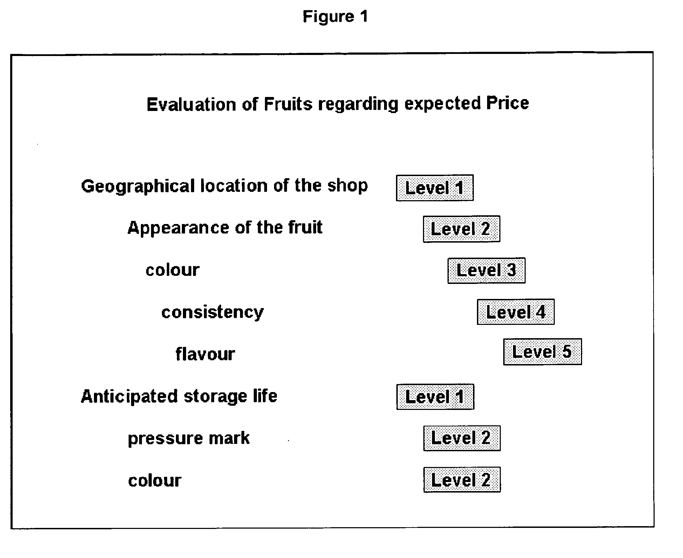

Figure 1 shows the method of the present invention ("Evaluation Tool") applied to the example ("Fruit Example").

### Overview of the invention

The present invention provides a variety of aspects, which are summarized below.

In principal, the method of the present invention enables to address various questions in different industry sectors related to the question of predicting success and failure of a compound or entity.

The data related to the different aspects to be addressed by this question are collected, categorized, and ranked with respect to the compound or entity to be evaluated in comparison to other compound or entities, respectively.

Possible entities are various products / services from different industries including (according to Anonymous, 2007a)
A - Agriculture, forestry and fishing
B - Mining and quarrying
C - Manufacturing including manufacture of chemicals and chemical products and Manufacture of basic pharmaceutical products and pharmaceutical preparations.
   The term "pharmaceutical" refers in a first aspect to pharmacy or to drugs and in a second aspect to a medicinal drug.
   Further types of Manufacture comprise: Manufacture of rubber and plastic products; manufacture of other non-metallic mineral products; manufacture of basic metals, manufacture of fabricated metal products, except machinery and equipment; manufacture of computer, electronic and optical products; manufacture of electrical equipment; manufacture of machinery and equipment n.e.c.; manufacture of motor vehicles, trailers and semi-trailers; manufacture of other transport equipment; manufacture of furniture, repair and installation of machinery and equipment, and other manufacturing)
D - Electricity, gas, steam and air conditioning supply
E - Water supply; sewerage, waste management and remediation activities (water collection, treatment and supply; sewerage; waste collection, treatment and disposal activities; materials recovery; remediation activities and other waste management services)
F - Construction (construction of buildings; civil engineering; specialized construction activities)
G - Wholesale and retail trade; repair of motor vehicles and motorcycles (wholesale and retail trade and repair of motor vehicles and motorcycles; wholesale trade, except of motor vehicles and motorcycles; retail trade, except of motor vehicles and motorcycles)
H - Transportation and storage (land transport and transport via pipelines; water transport; air transport; warehousing and support activities for transportation; postal and courier activities)
I - Accommodation and food service activities (accommodation; food and beverage service activities)
J - Information and communication (publishing activities; motion picture, video and television programme production, sound recording and music publishing activities; programming and broadcasting activities; telecommunications; computer programming, consultancy and related activities; information service activities)
K - Financial and insurance activities (financial service activities, except insurance and pension funding; insurance, reinsurance and pension funding, except compulsory social security; activities auxiliary to financial service and insurance activities)
L - Real estate activities
M - Professional, scientific and technical activities (legal and accounting activities; activities of head offices; management consultancy activities; architectural and engineering activities; technical testing and analysis; scientific research and development; advertising and market research; other professional, scientific and technical activities, veterinary activities)
N - Administrative and support service activities (rental and leasing activities, employment activities; travel agency, tour operator, reservation service and related activities; security and investigation activities; services to buildings and landscape activities; office administrative, office support and other business support activities)
O - Public administration and defence; compulsory social security
P - Education
Q - Human health and social work activities (human health activities; residential care activities; social work activities without accommodation)
R - Arts, entertainment and recreation (creative, arts and entertainment activities; libraries, archives, museums and other cultural activities; gambling and betting activities; sports activities and amusement and recreation activities)
S - Other service activities (activities of membership organizations; repair of computers and personal and household goods; other personal service activities)
T - Activities of households as employers; undifferentiated goods- and services-producing activities of households for own use
U - Activities of extraterritorial organizations and bodies

### Further embodiments of the invention

Besides the above mentioned method of the present invention a variety of additional embodiments are provided:
In one embodiment, a method is provided for determining whether a compound or entity has improved effects (or particular characteristics) compared to other members in a class of compounds or entities. In this embodiment "improved effects" means: higher grade of novelty, better safety, efficacy, less toxic effects, better benefit / risk ratio, less cost of goods, better strategic fit in the commercial environment.

In another embodiment, a method is provided for determining the best candidate in a class of compounds or entities. The profiling of a compound allows to compare this particular compound with other compound profiles regarding many different characteristics and therefore enables to identify the best compound among others.

The identification of the best candidate may be performed in a way that the different level 1 criteria are synergistically applied to come to a cumulative assessment of a compound or entity (see example 1).

In another embodiment, a method is provided for improving chances to select the best candidate in a class of compounds or entities. The profiling of a compound leads to the possibility to benchmark this particular compound characteristic. This procedure improves the probability to identify the best candidate in a class of compounds or entities.

In another embodiment, a method is provided of testing pharmaceutical compounds or compositions. The method may be used to identify certain strengths and weaknesses of a compound or combinations.

In another embodiment, a method is provided of evaluating pharmaceutical tests or biomarkers. The method may be used to comprehensively assess tests or effects of a compound or entities in the pharmaceutical field.

In another embodiment, a method is provided of selecting a pharmaceutical treatment. The method may help to identify the most suitable procedure to cure a patient.

In another embodiment, a method is provided of selecting a pharmaceutical treatment for a patient suffering from a disease, disorder or condition. The method may be used to identify the best cure for a particular individual. The individual patient may be identified using different methods (e.g. genetic markers and other markers) for personalized (i.e. individualized) medicine.

In another embodiment, a method is provided of performing a clinical trial.

The method may be used to simulate, prepare or support clinical trials and therefore to test a compound or an entity. In more detail, the method is suited to identify characteristics of pharmaceutical compounds which impact on the clinical trial design (e.g. regarding number of patients in a particular phase of a clinical trial, duration of treatment) based on the data collected for compounds with comparable, superior, inferior characteristics.

In another embodiment, a method is provided of increasing efficacy or chances of a clinical trial or a therapy. The method may help to identify special chances of a particular compound or an entity and therefore may help to reduce the risk of a compound or an entity in patients or animals or in *in vitro* tests.

In another embodiment, a method is provided of reducing risks of a clinical trial. The method may help to identify special hazards of a particular compound or an entity and therefore may help to reduce the risk of a compound or an entity in patients or animals or in *in vitro* tests.

In another embodiment, a method is provided of increasing safety of a clinical trial or a therapy. The method may help to identify special safety issues of a particular compound or an entity and therefore may help to reduce the risk of a compound or an entity in patients or animals or in *in vitro* tests.

In another embodiment, a method is provided of identifying the grade of knowledge about a compound. The method helps to profile both novel and known aspects of a compound or entity.

In another embodiment, a method is provided of treatment of a patient or a disease. The method helps to identify the optimal treatment for a particular patient or a disease.

In another embodiment, a method is provided of producing a pharmaceutical composition. The method may be used to identify the best compound for a pharmaceutical formulation and therefore form as an essential part of the process when producing a pharmaceutical composition. The pharmaceutical formulation may also comprise a combination of two or several compounds obtained with the evaluation tool of the present invention.

In another embodiment, a method is provided of identifying new treatment concepts for a particular disease. The method may be used to identify new or other relevant disease mechanisms to be targeted by new or existing compounds or entities, leading to new treatment concepts of patients.

All of these methods above may comprise one or more of the steps a) to c) of the method of the invention ("Evaluation Tool").

In addition the following embodiments directed to systems suitable to perform the above methods are provided:
a) A system for determining the best candidate in a class of compounds or entities. The invention displays comprehensive information in a way that it can be more easily be processed and is therefore assessable for decision making (e.g. through visualization of the profile of a compound or an entity).
b) A system for comparing candidates in a class of compounds or entities. The invention displays comprehensive information in a way that it can be more easily processed and is therefore assessable for decision making (e.g. through benchmarking compounds or entities).
c) A system for testing pharmaceutical compounds or compositions. The invention may be used as procedure to simulate the behaviour of a compound or an entity.
d) Use of system for determining the best candidate in a class of compounds or entities.
   Compound profiling and compound benchmarking lead to the identification of the best candidate.

### Detailed Evaluation Tool-Explanations

### A. Nature of data

There are two kinds of data, which may be used to evaluate a pharmaceutical compound in research and development:

### (1) Quantitative data:

There are various approaches to incorporate the comprehensive data obtained in drug discovery and development in the evaluation of compounds. One approach to organize this magnitude of data is the area of *"systems biology," which "[...] is the study of the behaviour of complex biological organization and processes in terms of the molecular constituents."* Applying this approach the respective data is made available for further analyses *"[...] through quantitative measurement, modelling, reconstruction, and theory."* (Kirschner, 2005)

### (2) Qualitative and semi-quantitative data:

Publicly accessible databases very often only provide plain texts (e.g. medline), semiquantitative data from different experiments or clinical trials (e.g. clinical scores).

However, it is frequently a problem to compare data from those various sources (e.g. proteome data vs. genomics data) or using different scales (e.g. different clinical scores) and to bring these data into perspective with the future fate of a drug in research and development.

The invention of the "Evaluation Tool" is intended to enable a comparison of the comprehensive publicly available data and proprietary data for different compounds and indications and make it accessible for decision-making. In principal, the "evaluation tool approach can be applied to all known disease entities (Cardiovascular Disorders; Clinical Pharmacology; Critical Care Medicine; Dermatologic Disorders; Ear, Nose, Throat and Dental Disorders; Endocrine and Metabolic Disorders; Eye Disorders; Gastrointestinal Disorders; Genitourinary Disorders; Gynaecology and Obstetrics; Haematology and Oncology; Hepatic and Biliary Disorders; Immunology; Allergic Disorders; Infectious Diseases; Injuries; Poisoning; Musculoskeletal and Connective Tissue Disorders; Neurological Disorders; Nutritional Disorders; Paediatrics; Psychiatric Disorders; Pulmonary Disorders; Special Subjects (for an overview reference is made to Anonymous, 2007b).

### B. Data entry

Data may be drawn from publicly available data sources such as databases (e.g. medline, etc.) or identified with the help of search algorithms in those databases (or optionally ontologies) (Bard, 2004). Additionally, not publicly available proprietary information may be put into the database (e.g. -omics data, clinical trial data).

In contrast to other databases the data are not entered in the form of plain text but in form of standardized and thus process able way. The data is stored in clearly defined data "boxes" (i.e. categories). Each of those boxes has a reference to the information source and date of publication (e.g. scientific publication, congress presentation, documents by the regulators, pointer to databases). The date of publication enables the reconstruction of the chronological sequence of the published (and not-published proprietary) data and will assist in the retrospective evaluation of compounds which have already been successfully developed or which failed at some point in discovery or development.

### C. Evaluation of data

To ensure the highest possible quality of the results each analysis will be divided into two parts: Firstly, a subset of the data may be used as training set to generate the hypothesis. Secondly, this hypothesis will be confirmed with the complimentary data part, the validation set.

To model the different characteristics of a compound in discovery and development and to predict the possible long-term success or failure of compounds Bayesian and Frequentist mathematical approaches will be applied.

Additionally, it is intended to analyse the data collected in the evaluation tool with the help of algorithms developed for so-called "network theory" [Definition: "A network is a set of items, which we will call vertices or sometimes nodes, with connections between them, called edges"; (Newman, 2003)]. Terms and algorithms like "centrality, closeness centrality, betweenness centrality, eigenvector centrality" and others have been applied to analyses in "Network biology" (Newman, 2003; Barabasi and Oltvai, 2004). The results of these network analyses will even be evaluated with the help of Frequentist and Baysian mathematical approaches. Correlation and Cluster analyses will be performed.

### D. Objectives of the evaluation

As a result of the evaluation process a scientific "compound profile" is prepared which covers the different characteristics of the compound. Based on this compound profile a particular compound can be compared to with other compounds ("benchmarking of compounds").

Similarly, to "compound profiling" and "compound benchmarks" indications will be profiled and put into perspective with other indications ("indication profile" and "indication benchmarks"). The short-term objective is to compile the comprehensive compound profiles (retrospective analyses). As a long-term objective success criteria of compounds in discovery and development will be prospectively assessed.

Additionally, the identification of opportunistic indications for a given compound is possible. Moreover, various scenarios for research and development of a pharmaceutical compound may be compared including the comparison of sensitivity and specificity of different study designs.

Moreover, other aspects in research and development may be compared (e.g. costs, numbers of treated patients, timelines). Therefore, the approach can be used to optimize the development plan. Even the preparation of a pharmacovigilance plan and analysis of safety data can be supported. Furthermore, the obtained data can be used for marketing purposes and for licensing activities, investment decisions (investors, analysts), technological developments (early detection of new methodologies applied, supporting technology suppliers/supply chain companies to plan capacities and own developments in this area).

### E. Display of the analysed results

A compound profile consisting of different compound characteristics (e.g. dealing with safety and efficacy, novelty of approach or compound profile) will be formed (compound profiling). Subsequently, the compound characteristics will be compared to other drugs in the same and other indications (compound benchmark). The results of these analyses will be graphically displayed. This allows a swift optical comparison of different compounds on the basis of various characteristics. Additionally, strengths and weaknesses of compounds may be more easily identified. In long-term, prospectively collected data using the various compound characteristics will be used to predict the probability of success of a compound. This may finally lead to the assignment of a cumulative "probability of realization" score for each compound to be evaluated at different steps of drug discovery and development.

### Example 1: Exemplification of the "Evaluation Tool" with the help of the "Fruit Example"

Fruits have to be evaluated before they can be sold or bought. As a basis data is collected regarding the price, which can be achieved on the market. For this purpose an algorithm has to be identified.

Following the method of the present invention ("Evaluation Tool") the fruits will be evaluated.

In the simplest mode, there is only a retrospective evaluation of this year's harvest. However, more detailed evaluations are possible allowing future prognosis regarding the revenue of future harvest in upcoming years will be possible.

The following rules for filling of the "evaluation tool" have to be complied with:
Fruits have distinct characteristics (data). According to these characteristics the fruits are put into distinct boxes (categories). The boxes are than put into additional boxes (Level 1 - ∞ categories) (please also refer to figure 1).

Initially, a fruit is put into exactly one box. This box in turn will be put into other boxes (in case that there is a natural or calculated hierarchy for those boxes, s. above).

Boxes may also be put in other boxes of a higher level (in case that there is a natural or calculated hierarchy for those boxes).

In one example, the box "flavour" has several boxes with a descriptive term for "flavour" (sweet, sour, bitter, ...) or the combination of several descriptive terms.

Boxes can be put into lower level boxes (e.g. level 3) or into higher level boxes (e.g. Level 2) on the basis of two different principles:
1. natural hierarchy
2. calculated hierarchy

The same boxes can appear in different hierarchies (example: "colour" appears under "anticipated market success" as Level 3 and under "anticipated storage life" as Level 2).

To assign fruits with regard to the characteristics put under "anticipated storage life" the fruits are not put into baskets but an alternative labelling system is used (e.g. dots of different colours are painted on the fruits).

Primarily, there is a qualitative allocation of the particular boxes with the help of naturally or a calculated hierarchy. Only few boxes cannot be sorted in a hierarchical way and will appear as Level 1 boxes (s. below).

### 1. Natural hierarchy

In the evaluation process a fruit is being evaluated regarding its characteristics: In a first step, a "natural evaluation cascade" will be build according to the taxonomy of the respective term. Taxonomy is the classification of all items into conceptual categories, which belong to hierarchy L₁ categories. For each taxon there are sub-taxa: First the category "geographical location of the shop" will be evaluated (Level 1). Second, the Level 2 category "appearance of the fruit" will be evaluated. This is a natural hierarchy because a costumer will only be able to evaluate the ""appearance of the fruit" if the geographical location of the shop allows him to evaluate the fruit itself at all.

During the next steps different aspects of the "appearance of the fruit" are evaluated by the costumer. The sequence of these evaluation steps follows a "natural hierarchy": First the customer sees the fruit ("colour", Level 3), than the costumer touches the fruit ("consistency", Level 4), than the costumer takes a smell at the fruit ("flavoue", Level 5). If the costumer is not satisfied with the "colour" of a fruit he won't perform the further evaluation steps "consistency" and "flavour". In this respect the Level 2 box "colour" is more important than the box "consistency" which is Level 3.

The step "flavour" will not be made until the step "colour" has been successfully performed. The step "colour" is followed by the step "consistency".

"Colour" is Level 3 and therefore below the box "appearance of the fruit" (Level 2).

"Consistency" is situated on Level 4 and therefore one level below the "colour"-box (Level 3).

"Flavour" is situated on Level 5 and therefore one level below the "consistency-box" (Level 4).

### 2. Calculated hierarchy

If a fruit is being evaluated regarding its "anticipated storage life" no "natural evaluation cascade" is evident as in the case of "appearance of the fruit". Therefore, fruits will be sorted on the basis of the two Level 2 characteristics "pressure marks" and "colour" into boxes. The meaning of the evaluated characteristics "pressure marks" and "colour" for the "anticipated storage life" will have to turn out later on the basis of the actual storage life. Later on, a hierarchy can be build: The higher the statistical correlation or other statistical evaluations (s. also above), the higher the level of the characteristic is, it will be sorted in.

### 3. Characteristics that cannot be sorted into a box of the hierarchy

Not all boxes can be sorted into a box of the hierarchy. Therefore, two Level 1 criteria for the anticipated revenue are identified: Anticipated market success, and anticipated storage life. Those two Level 1 criteria cannot be put further in an hierarchical order, since they do not have anything to do with each other (this is at least assumed for the presented example), i.e. those boxes will be put next to each other on the same level.

### 4. Evaluation

The fruits are evaluated on the basis of different characteristics, which help to predict their probability of being sold on the fruit market (e.g. anticipated market value, anticipated storage life). In other cases the combination of different characteristics of lower levels (e.g. geographical location of the shop, "appearance of the fruit") will help to predict later success on the market.

In some cases information regarding the fruit might be missing. Using the above described method the relevance of each singular missing information / data for predicting the probability of success or failure can be identified. Therefore, the necessity to generate the respective data can be estimated to prioritize the different putative experiments and other measures.

### 5. Profiling

The sum of all different (in part hierarchically sorted) categories, which describe a particular fruit is called the "profile of the fruit" and the process of achieving this "profile" is called "profiling".

### 6. Benchmarking

Two different fruits can be compared with each other regarding a particular feature (e.g. colour). The relative position of a particular fruit in comparison with other fruits is called benchmark. The process of creating a "benchmark" is called "benchmarking".

## Claims

1. A method for comparing and evaluating candidates in a class of compounds or entities, the method comprising:
a) Performing compound profiling by defining various categories and assigning plural defined categories to individual compounds or entities and characterizing and evaluating compounds or entities according to said categories;
b) Performing compound benchmarking by comparing results within the same category of one compound with results of the same category of other compounds or entities and generating compound benchmarking for different categories based on the outcome of the comparison;
c) Generating a benchmark representation including combining different compound benchmarks for different categories.

2. The method of claim 1, for determining whether a compound or entity has one or more improved effects compared to other members in a class of compounds or entities, further comprising:
d) Determining the compound or entity with one or more improved effects based on the results of b) and/or c).

3. The method of claim 1 for determining the best candidate in a class of compounds or entities, further comprising:
d) Determining the best candidate in a class of compounds or entities based on the results of b) and/or c).

4. A method of producing a pharmaceutical composition, comprising the steps a) to c) of claim 1 and further comprising:
a) Performing compound profiling by defining various categories and assigning plural defined categories to individual compounds or entities and characterizing and evaluating compounds or entities according to said categories;
b) Performing compound benchmarking by comparing results within the same category of one compound with results of the same category of other compounds or entities and generating compound benchmarking for different categories based on the outcome of the comparison;
c) Generating a benchmark representation including combining different compound benchmarks for different categories;
d) Identifying the best pharmaceutical compound and producing same in the form of a pharmaceutical composition.

5. The method of claim 1, wherein step a) of claim 1 comprises:
α) Collecting data d₁(i), d₂(i), ... dₙ(i), with n = 1 ... n = ∞ for each member mᵢ of a class of compounds or entities C(mᵢ), with C(mᵢ) and 0< i< ∞;
β) Putting data d₁(i), d₂(i), ... dₙ(i) into different categories S₁(i), S₂(i), .... Sₙ(i), with n = 1 ... n= ∞, wherein
β1) for each member mᵢ among the categories S₁(i), S₂(i), .... Sₙ(i) particular categories are selected which are sorted under the first level of hierarchy L₁ according to the taxonomy of hierarchy L₁, wherein taxonomy is the sorting of categories and sub-categories L₁, L₂, ... L_{∞} below a particular L₁ category and all categories below a particular L₁ level category conceptually belong to this L₁ category;
β2) for each member mᵢ among the remaining categories S₁(i), S₂(i), .... Sₙ(i) categories are selected, which are sorted under the other levels of hierarchy sub-levels L₂,L₃ ... L_{∞} according to the taxonomy of the respective level L₂, L₃ ... L_{∞};
β3) for each member mᵢ among the still remaining categories, which cannot be sorted based on the taxonomy are correlated with the overall success or failure of the compound or entity to be evaluated and are grouped into additional sub-levels L₂,L₃ ... L_{∞}, wherein
overall success or overall failure is defined as a function of all members mᵢ of a class of compounds or entities C(mᵢ), with C(mᵢ) and 0< i< ∞, which fulfil particular predefined criteria for success or failure,
overall success is defined as the achievement of a predefined objective or goal, and
overall failure refers to the state or condition of not meeting predefined objective or goal and is defined as the opposite of success, and
for the data for each member mᵢ and each first Level L₁ a ranking Rᵢ,L₁ is established as follows: R_{¡},L₁ = {dj(L₁), dk(L₂), dl(L₂), ...}, with j,k,j are integers equal to or greater than 1;
wherein step b) of claim 1 comprises:
γ) Comparing or sorting the members mᵢ of the class of compounds or entities C(mᵢ), comprising:
γ1) determining a probability of success p₁(m_{¡}) for each member mᵢ of a class of compounds or entities which is a function of all d_{¡}(L₁) of the first level L₁ hierarchy;
γ2) determining further probabilities of success pₓ(mᵢ) for each member mᵢ of a class of compounds or entities which is a function of all dᵢ(Lₓ) of the x-^{th} level L₁ hierarchy;
γ3) comparing each member mᵢ of the class of compounds or entities C(mᵢ) with each other member mⱼ of the class of compounds or entities C(mᵢ) within the same L₁, L₂,L₃... L_{∞} by comparing probabilities pₓ(m_{¡}) and pₓ(mⱼ);
γ4) optionally sorting all members mᵢ based on the order of probabilities p₁ and pₓ, wherein the probabilities p₁ are used in a first sorting step, the probabilities pₓ are used in a x-^{th} sorting step, wherein if there are more than one data and probabilities of the first level L₁ for each ranking R a sorting step is performed;
δ) Comparing all data d₁(i), d₂(i), ... dₙ(i) of a compound or entity mᵢ within the same L₁, L₂,L₃... L_{∞} as a function of R_{¡},L₁ = {dj(L₁), dk(L₂), dl(L₂), ...}, which means a comparison of each compound with each other compound regarding the same category S₁(i), S₂(i), .... Sₙ(i) is enabled, wherein the combination of different R_{¡},L₁ = {dj(L₁), dk(L₂), dl(L₃), ...} in different L₁, L₂,L₃...L_{∞} enables to characterize the various facets of a compound or entity, wherein
δ1) the probability of success or failure is calculated for each category S₁(i), S₂(i), S₃(i), ... S_{∞} within L₁ regarding each member mᵢ of a class of compounds or entities C(mᵢ), with C(mᵢ) and 0< i< ∞ and compound profiling is the combination of different probability of success or failure for each member mᵢ of a class of compounds or entities C(mᵢ), with C(mᵢ) and 0< i< ∞ at each L₁, respectively;
δ2) additional information can be added by calculating the probability of success or failure for each category S₁(i), S₂(i), .... Sₙ(i) at each level L₂,L₃ ... L∞; and δ3) additional information may be added by calculating the probability of success or failure for the combination of different categories S₁(i), S₂(i), .... Sₙ(i) below different L₁, L₂,L₃ ... L_{∞};
ε) Optionally selecting a optimal member mₘₐₓ out of the class of compounds or entities C(mᵢ) based on the basis of one or more of the probabilities of success obtained in steps δ1) and/or δ2) and/or δ3), and
optionally providing a row of members m_{sec}, m_{third}, ... out of the class of compounds or entities C(mᵢ) based on the basis of one or more of the probabilities of success obtained in steps δ1) and/or δ2) and/or δ3), wherein m_{sec}, m_{third}, ... are the second and third, ... optimal members; and
ζ) Identifying the possibility to improve the probability of success / reduce the probability of failure by adding additional data to each member mᵢ of a class of compounds or entities C(mᵢ), with C(mᵢ) and 0< i< ∞.
